# EUROPEAN PATENT APPLICATION

(11) **EP 1 840 567 A1**
(43) Date of publication of application: **03.10.2007**
(21) Application number: 07251409.4
(22) Date of filing: 30.03.2007
(51) Int. Cl.: G01N 33/28, G01N 30/96

(54) **Crude oil screening process**

(30) Priority: 30.03.2006 GB 0606387
(71) Applicant: Oil Plus Limited, Newbury, Berkshire, RG14 5TR (GB)
(72) Inventor: Smith, Patrick Colin, c/o Oil Plus Limited, Newbury Berkshire, RG14 5TR (GB); Turner, Michael, c/o Oil Plus Limited, Newbury Berkshire, RG14 5TR (GB); Smith, Desmond, c/o Oil Plus Limited, Newbury Berkshire, RG14 5TR (GB); Roberts, Steven, c/o Oil Plus Limited, Newbury Berkshire, RG14 5TR (GB)
(74) Representative: Copsey, Timothy Graham

(57) **Abstract**

A method for the quantitation of the naphthenic acid content in a hydrocarbon composition, comprising (i) separating naphthenic acid from the hydrocarbon composition; (ii) applying the separated naphthenic acid to a macro reticular ionexchange resin additionally comprising transition metal ions co-ordinated with an amine or ammonia in which the metal ion amine/ammonia co-ordinated complexes are bound to the surface of the resin; (iii) washing the column; (iv) eluting the naphthenic acid-metal ion complexes from the column; and (v) quantitating the concentration of naphthenic acid-metal ion complexes present in the eluate from the column.

## Description

The present invention relates to a method of screening crude oil to determine to amount of high molecular weight naphthenic acid present. The method provides a means by which crude oil can be screened to assess quality and also purified to remove such impurities

There are an increasing number of new oil reservoirs being discovered and developed in recent years that yield heavier oil types (i.e. oils with API gravities of between 15 to 25°). With sustained higher oil prices such oilfields have become more viable. One common trait for these oils is that they show high total acid values and significant naphthenic acid contents. In refineries these oils are known as High Acid Crude (HAC) feedstocks. The actual Total Acid Number (TAN) values (mg KOH / g oil) of these oils does not correlate at all with their risk of forming naphthenate solids or other soap types during reservoir fluid production in oilfields. These soap materials are an increasingly more common and very severe flow assurance problem being encountered worldwide [1 to 6]. For example, there are rare cases of 35° API oils of 0.10 TAN that can yield high amounts of naphthenate solid precipitates during production [5]. Alternative ways of defining the relative organic acid fraction of crude oils is a real need in the oil industry, for both the upstream and downstream industries.

Within the last five or more years there has been an increasing number of oilfields produced with varying amounts of naphthenic acids [1 to 6], or rather, the understanding in the oil industry has reached a level where people are now confident in publishing papers in this area. In truth, naphthenic acid problems have been experienced for at least fifty years in the oil industry, mainly manifesting themselves as stable emulsions and dirty disposal water problems in oilfields, e.g. in Southeast Asia such problems have been observed since the early 1960s. These problems were associated with the formation of strong surfactant properties of soaps of organic acids [1, 4, 5, 6 & 8 to 11]. It is noted that as existing crude oil reservoirs are becoming depleted, the newer reservoirs being discovered now, are ones that in general are classed as immature reservoirs and are commonly biodegraded oils that have lost many components (e.g. alkanes and waxes) and thus the naphthenic acids are found to occur in increasing concentrations [2, 8, 9].

By definition a naphthenic acid is a monobasic carboxy group attached to a saturated cycloaliphatic structure. It has been accepted in the oil industry that all organic acids in crude oil are called naphthenic acids. Naphthenic acids in crude oils are mixtures of low to high molecular weight organic acids. Such acids can be very water soluble to oil soluble depending on their molecular weight, on the process temperatures their parent fluids are subjected to, on the salinity and pH of their parent waters and finally on their parent fluid processing pressures [1, 2, 5 & 6].

Naphthenic acid nomenclature was always known in peer groups as a misnomer as it implied unsaturation due to the "enic" in its name. Naphthenic acids were discovered over 100 years ago [12]. The old classification indicates that naphthenic acid is a monobasic carboxy group attached to a saturated cycloaliphatic structure [12]. It has been quoted that all organic acids in crude oil are called naphthenic acids but this may not be true [12]. This is simplistic, as acetic acid is not recognized as a naphthenic acid. Heavy Californian crude oils are known to yield naphthenic and naphtheno - aromatic acids, for example [13, 14, 15].

Naphthenic acids in crude oils are mixtures of low to high molecular weights species varying from approximately 100 to >1300 molecular weight (MW), as shown by various analyses, including mass spectrometric (MS) and Fourier Transform Ion Cyclotron Resonance (FTICR) analyses [6, 7]. In the water phase, naphthenic acids can cause stable reverse emulsions. In the oil phase with residual water, these acids have the potential to react with a host of minerals, which are can neutralize the acids to form insoluble salts.

The main reaction product found in actual oilfields is the calcium naphthenic acid soap, known to the oil industry as calcium naphthenate. Analyses of various actual soaps samples from oilfields around the globe indicates that they are often a mixture of one or more soap (including calcium, magnesium, sodium, potassium, iron, and aluminium), with occluded silica/sand, mineral scales, iron hydroxides/oxides and sulphides, formation clay, mud residues e.g. bentonites, flocculated asphaltenes, paraffin waxes, resins and treating chemicals [5, 6, 8]. These soaps and other occluded particles have been known under real conditions for a long time [16, 17]. These adhere to oil water interfaces and can become finely dispersed within the water itself. The oil industry to date has solved the emulsion problems caused by naphthenate soaps using acidic demulsifiers, called "pad busters" or interface draw-offs to separate slop treatments [2 to 6 & 8]. The reverse emulsions in oils and in dirty oilfield water are being treated with water clarifiers, most of these being the cationic water clarifiers [5, 8].

The design of typical production processes and the decreases in fluid pressures from wells to separation trains affect the bicarbonate, carbon dioxide and pH equilibrium in produced waters. This provides a driving force for the formation of calcium naphthenate soaps [5, 6, 18, 19]. These calcium naphthenate soaps (including contaminants), can deposit across the oil process system from wellheads, subsea flowlines, separators, chemelectrics, heat exchanger tubes to oil filters and even within oil storage tanks. These deposits can congeal to hardened cement-like structures requiring frequent system shutdowns and physical clean-up periods costing millions of dollars in maintenance costs and lost production [5].

Calcium naphthenate dispersed in crude oils to refineries can sludge out in terminal tanks and cause fouling in lines, desalters and heat exchanger units. In refineries most analyses of the naphthenic acid fractions are important due to implications in the corrosion process [9, 17, 20, 21]. Results are reported on the medium molecular weight (250 - 450 MW) naphthenic acids to enable prediction of overheads corrosion/neutralizer rates [22] and the amount of caustic and/or ammonia double decomposition required to combat naphthenic acids and soaps prior to desalters and distillation units. There is no distinction between high and low molecular weight naphthenic acids in refineries. Prior to the ARN acid discovery [7], mass spectrometric analyses were concentrating in the low molecular weight acids (LMWA) area of up to 320 MW [18].

In the water phase, naphthenic acids and their soaps have been known to cause stable reverse emulsions (i.e. oil droplets in a continuous water phase) in some production systems [5]. In the oil phase with residual water, these acids have the potential to react with a host of minerals, which are capable of neutralizing the acids. The main reaction product found in practice is the calcium naphthenic acid soap (the calcium salt of naphthenic acids). These often become insoluble salts and form solids material that can plug production systems, eventually causing system shutdowns [1 to 6]. Analysis of these collected soap solids has proven that calcium soap is often a major component. Soap agglomerations in wells and separation trains can generate a mixture of magnesium, sodium, potassium, iron, and aluminium soaps with occluded formation-derived sand, silt and clays, mineral scales, iron scales, precipitated asphaltenes, resins, petroleum waxes, mud components and treatment chemicals [5,6].

Several gas chromatography and combined expensive mass spectrometric analytical procedures have not so far been able to give acceptably accurate quantitative determinations of the precursor problematic acids contents, in oils prone to generating these soaps. Other methods involve use of a pilot plant to determine the organic scale-soap probability under a combination of synthesised process conditions, typically using significant volumes of aged 'stabilised' (degassed) crude oil samples [6].

A further development is the identification of a newly defined set of naphthenic acids, called the ARN acids, typically of mass over charge, m/z, of 1230 amu (atomic mass units, or Daltons), which have been identified as being present in significant quantity in many oilfield naphthenate deposits and crude oils [5, 6, 7]. It has been proposed that the specific acid types are one of the main precursor acids present in crude oils that must be available to form the calcium and other naphthenate soap solids [6, 7]. The ARN acid structures have yet to be fully defined [7] but they are important acids and they do form a proportion of the acids isolated by the methodology described in this patent.

Previous and current techniques for analysis of naphthenic acids do not examine the relative amount of high molecular weight naphthenic acids. Expensive techniques are employed which are not valuable on a routine basis or practical in field conditions. Chromatographic techniques, including GC-MS [23], MS [15], resolve the naphthenic acids into hundreds of peaks. Analysis of the naphthenate soaps by a Norwegian research group found a strange high molecular weight acid species, designated ARN acids, 1230+ MW, m/z, z=1, which is tetraprotic and also gives a 615 m-2H/z (z=2) signal [6, 7]. These ARN acids were qualitatively detected as the major components in various naphthenate deposits. Previous researchers emphasised the generalised 250-450 MW areas for naphthenic acids [18, 22]. Thus the ARN acid discovery was a revelation in the naphthenate soap story.

Many laboratory investigations have been performed on naphthenic acids and naphthenate solid soaps within the last five years. There are publications from Total [1, 2, 10], Conoco Phillips and Statoil [7], ExxonMobil [22], Heriot Watt University with Oil Plus [6] and university research students Brandl O. and Havre T.E. [19, 24] of NTNU, Professor J. Sjoblom. Experimental work ranged from normal reaction of naphthenic acids with brine to form soaps [1] performed by Rousseau et al, emulsion studies looking at the effects of naphthenic acids [18], naphthenic acids on silica gel (25), Near Infra red work [26] and LC-MS analysis, after anion exchange, the Acid IER Method (10), to name a few. None of these have correlated a ranking table or prediction of good oils versus bad oils with respect to their potential to form naphthenate soaps. The ARN acids discovery [7] is the building block to further work and presents the opportunity to use this, in a more realistic methodology, for problematic naphthenate soap predictions.

When evaluating literature search results for most published approaches to naphthenic acids quantification or isolation, it can be seen that there is a practical problem to be solved with respect to the significance of the amount of the naphthenate precursor ARN-type acids present in oils, that would lead to actual naphthenate soap deposition problems within oilfield production systems. One major question is how much of these cyclic acids would need to be present in a crude oil for that oil to be designated a problematic naphthenate-forming oil.

The very instrumental reliant liquid chromatography-mass spectrometry (LC-MS), electrospray mass spectrometry (ES-MS) and gas chromatography-mass spectrometry (GC-MS) techniques employed thus far (start of 2006), are not ideal for routine on-site work in oilfield laboratories [6]. There are no published results on the analytical ranking of the special properties of crude oils relative to known oils with problematic naphthenate soap formation tendency and to oils with no naphthenate soap formation tendency.

There is therefore a need for a process that overcomes the problems inherent in the methods used to date.

According to a first aspect of the invention, there is provided a method for the quantitation of the naphthenic acid content in a hydrocarbon composition, comprising
(i) separating naphthenic acid from the hydrocarbon composition;
(ii) applying the separated naphthenic acid to a macroreticular ion-exchange resin additionally comprising transition metal ions co-ordinated with an amine or ammonia in which the metal ion amine/ammonia co-ordinated complexes are bound to the surface of the resin;
(iii) washing the column;
(iv) eluting the naphthenic acid-metal ion complexes from the column; and
(v) quantitating the number of naphthenic acid-metal ion complexes present in the eluate from the column.

In its broadest sense, the naphthenic acid content of a hydrocarbon composition is a reflection of the content of all of the organic acids present in the sample, i.e. carboxylic acids. The term naphthenic acid may also be used more narrowly to describe certain specific organic acid components of crude oil as follows.

Single and multiple fused cyclopentane and cyclohexane rings where the carboxylic acid group is attached to an aliphatic side chain or to a cycloaliphatic ring may be described a naphthenic acids

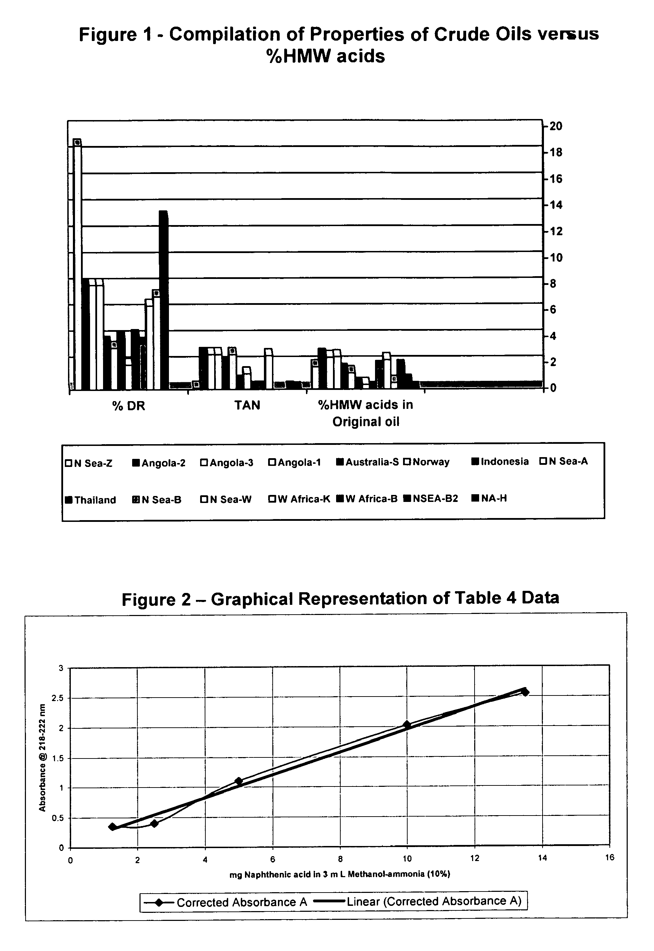

The American Petroleum Institute (API) definition for naphthenic acids includes structures with single and multiple fused cyclopentane and cyclohexane rings as illustrated in Fig. 1, where the carboxyl group may be attached either to the aliphatic side chain (preferably) or to the cycloaliphatic ring (API 2003).

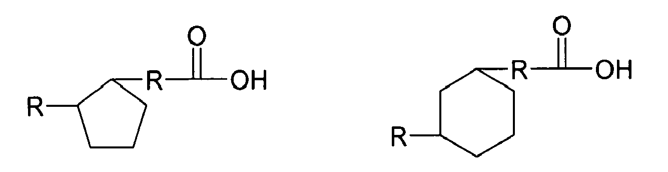

Based on these assumptions, it is possible to represent naphthenic acids by the formula, CₙH_{2n+Z}O₂, where, n is the carbon number and Z represents a homologous series. Z can be 0 or a negative even integer (-2, -4, etc) and reflects the hydrogen loss or deficiency which occurs when a ring is included in the acid structure. Thus, Z = -2 equals one cycloaliphatic ring, Z = -4 equals 2 cycloaliphatic rings, etc. More than one isomer will typically exist for a given Z group.

As noted above, the term naphthenic acid may also used to identify *all* of the carboxylic acid content of a crude oil which can include alkyl substituted acyclics (including "fatty" acids), aromatic acids, carbazoles and isoprenoid acids. More complex acid structures with two, three and even four carboxylic groups (tetrameric acids) have been identified as well as structures containing heteroatoms (O, O₄, S, OS, O₂S, O₃S, N, NO, NO₂, N₂O) have been found in certain crude oils.

The specific acids may be tetrameric acids, in the molecular weight range of 1227 to 1235 Da. The acids homologous series corresponds to empirical formula of C₈₀H₁₃₈O₈, C₈₀H₁₄₀O₈, C₈₀H₁₄₂O₈, C₈₀H₁₄₄O₈ and C₈₀H₁₄₆O₈ with double bond equivalences (DBE) ranging from 12 to 8, indicating 8 to 4 rings in the hydrocarbon skeleton, respectively.

References to naphthenic acid include naphthenate and *vice versa* unless the context clearly specifies otherwise.

Hydrocarbon compositions, which can be analysed by a method of the invention, include crude oil, or partially purified crude oil, or an oil or substance obtained from crude oil following subsequent crude oil distillation, for example petroleum, kerosene, or paraffin. The method may be practised on samples obtained from crude oil directly, or from sludges, oil deposits, oil emulsions, or tars which have been prepared for sample analysis. Preparation for sample analysis may include appropriate steps to remove particulate and/or solid matter, excess water or other impurities. Excess water may be removed by a process of alternate heating and cooling of the sample, followed by centrifugation to remove the water. Alternatively, the water may be removed manually. The heating process may be carried out in an inert atmosphere, e.g. under nitrogen or helium or other inert gases.

The separation of naphthenic acid from the hydrocarbon composition may be achieved by means of a process such as dialysis. A dialysis procedure uses a membrane selected to be permeable to certain substances so as to permit a separation of the substance of interest from the remainder of the substances present. In the present case, the membrane may be permeable to low molecular weight naphthenic acids and/or other low molecular weight impurities. A preferred dialysis procedure is the closed sack distillative reflux dialysis method which comprises extraction by dialysis under refluxing solvents. In such a method, the sample may be placed in a membrane dialysis sack (a.k.a. finger cot). Sack details are as follows. Supplier DEK Northern Europe, Granby Industrial Estate, Weymouth, Dorset DT4 9^{TH}, UK, Part no 173035. Sack/finger cot specifics are - Anti-Static latex ISO 5 (Class 100 7C), with the proprietary anti-static agent yielding average surface resistance of 5 x 10 12 Ohms Per square unit. Washed powder-free 3 ml latex cleanroom processed and packaged in ESD safe inner bags and covering the small. Medium, large and extra large sizes (Product Numbers PN-173033, 173034, 173035 and 173057). During the distillation phase of the procedure, the membrane sacks may suitably be placed into a holder or a basket.

At the conclusion of the dialysis procedure, the solvent dialysate surrounding the dialysis sack is removed. The dialysis residue containing the higher molecular weight naphthenic acid content is then removed for application to the macroreticular ion-exchange resin. The sample may be further concentrated, or otherwise prepared for application onto the resin. The sample may be dissolved and volumetrically prepared in organic solvents.

Suitable solvents may be a straight chain or branched C1-C10 alkane. The term C₁-C₁₀ includes the groups methyl, ethyl, propyl, butyl, pentyl, hexyl, heptyl, octyl, nonyl, and decyl. The alkane may be pentane, hexane, and or heptane. Preferably, hexane may be used as the dialysis extraction solvent.

Suitably, the resin is packed into the form of a column so as to assist the analysis of the sample. Where the resin is packed into the form of a column, the sample is loaded onto the column at one end.

The macroreticular ion-exchange resin may be a macroporous ion-exchange resin, suitably composed of a polymeric material. The polymeric material may be optionally cross-linked. For example, a suitable macro-reticular resin may be composed of cross-linked styrene divinylbenzene copolymers. A preferred type of such cross-linked styrene divinylbenzene copolymer macroreticular resins is Amberlyst 15® and/or Amberlyst A31 (Rohm & Haas).

The macroreticular resin is modified in that it comprises transition metal ions co-ordinated with an amine or ammonia in which the metal ion amine/ammonia co-ordinated complexes are bound to the surface of the resin. Suitably, the transition metal ion is supplied in the form of a water-soluble salt, which can be subjected to admixture with the amine or ammonia to co-ordinate the transition metal ion.

Suitable water-soluble salts include, but are not limited to, sulphate, nitrate, carbonate, halide (i.e. fluoride, chloride, bromide, iodide). The resultant complex can be formed in situ on the column where the amine or ammonia is already bound to the column.

The transition metal ion may be one or more the following: a Period 4 transition metal ion, a Period 5 transition metal ion, a Period 6 transition metal ion or a Period 7 transition metal ion. Alternatively, the transition metal ion may be a Group 3 (III B), Group 4 (IV B), Group 5 (V B), Group 6 (VI B), Group 7 (VII B), Group 8 (VIII B), Group 9 (VIII B), Group 10 (VIII B), Group 11 (I B), or Group 12 (II B) transition metal ion.

Period 4 transition metal ions include: Scandium (Sc), Titanium (Ti), Vanadium (V), Chromium (Cr), Manganese (Mn), Iron (Fe), Cobalt (Co), Nickel (Ni), Copper (Cu), Zinc (Zn). Period 5 transition metal ions include: Yttrium (Y), Zirconium (Zr), Niobium (Nb), Molydenum (Mo), Ruthenium (Ru), Rhodium (Rh), Palladium (Pd), Silver (Ag), Cadmium (Cd). Period 6 transition metal ions include: Lutetium (Lu), Hafnium (Hf), Tantalum (Ta), Tungsten (W), Rhenium (Re), Osmium (Os), Iridium (Ir), Platinum (Pt), Gold (Au).

Group 3 (III B) transition metal ions include Scandium (Sc), Yttrium (Y), Lutetium (Lu). Group 4 (IV B) transition metal ions include Titanium (Ti), Zirconium (Zr), Hafnium (Hf). Group 5 (V B) transition metal ions include Vanadium (V), Niobium (Nb), Tantalum (Ta). Group 6 (VI B) transition metal ions include Chromium (Cr), Molydenum (Mo), Tungsten (W). Group 7 (VII B) transition metal ions include Manganese (Mn), Rhenium (Re). Group 8 (VIII B) transition metal ions include Iron (Fe), Ruthenium (Ru), Osmium (Os). Group 9 (VIII B) transition metal ions include Cobalt (Co), Rhodium (Rh), Iridium (Ir). Group 10 (VIII B) transition metal ions include Nickel (Ni), Palladium (Pd), Platinum (Pt). Group 11 (I B) transition metal ions include Copper (Cu), Silver (Ag), Gold (Au). Group 12 (II B) transition metal ions include Zinc (Zn), Cadmium (Cd).

Preferred transition metal ions may include metal ions selected from the group consisting of Copper (Cu), Silver (Ag), Nickel (Ni), Cobalt (Co), Iron (Fe), Manganese (Mn), Molybdenum (Mo), Chromium (Cr), and Platinum (Pt). More particularly preferred transition metal ions may be Copper (Cu), Nickel (Ni) or Molybdenum (Mo).

In one embodiment of the invention, the transition metal ion is not added to the column but rather the complex is prepared on the column, so the reaction to form the complex takes place on the column.

The transition metal ion is co-ordinated with an amine or ammonia. The transition metal ion is suitably adsorbed on to the column and the amine or ammonia is then suitably fixed on to the metal to form the appropriate metal/ammonium or metal/amine complex in situ. For example, where the metal is copper, the use of ammonia will lead to the formation of the corresponding cuprammonium complex and the use of an amine will lead to the formation of the appropriate cupramine complex.

The amine may be an amine of general formula: 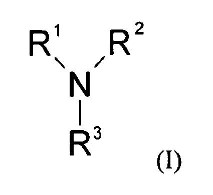 in which R¹, R² and R³ may each independently be Hydrogen (H), or a C₁-C₆ straight chain or branched alkyl group, optionally substituted with Oxygen (O). The term C₁-C₆ includes the groups methyl, ethyl, propyl, butyl, pentyl, and hexyl.

Where one or more of R¹, R² and R³ are an alkoxy group it may comprise from 1 to 14 alkoxylated groups, selected from ethylene oxide, propylene oxide or butylene oxide and mixtures thereof.

The amine may be a linear, branched chain or fused-ring system amine. For a fused-ring system amine, either R¹ and R², or R² and R³ or R³ and R¹ can be joined together to form a ring which may be saturated or unsaturated.

The amine may be a primary, a secondary or a tertiary amine. Suitable amines include methylamine (CH₃NH₂), ethylamine (C₂H₅NH₂), ethylene diamine (NH₂CH₂CH₂NH₂), pyridine, morpholine, cyclohexamine, or benzylamine. Where all groups R are Hydrogen, the general formula will represent ammonia.

A coordinated complex of the transition metal ion and the amine of general formula (I) or ammonia may be formed by in situ on the macro-reticular resin by adsorption entrapment. Macroreticular resins, e.g. in the form of a macroreticular resin column are specifically designed to trap metal ions.

After loading the sample onto the column, it suitably allowed to equilibrate to permit the formation of naphthenic acid complexes with the bound transition metal ion amine/ammonia complex. After a sufficient period of time has elapsed, the column is then washed to remove unbound sample and also to remove uncomplexed transition metal ion (impurities).

The column may be washed with solvents such as xylene and/or heptane which may assist in removal of heavy oil residues. Xylene and/or toluene may also be used for this purpose. A final wash of isopropanol may be used to swell the resin to assist in washing the column of excess residues.

Washing the column may therefore comprise one or more of the following steps:
- wash with xylene / heptane mixture
- wash with xylene / toluene mixture
- wash with isopropanol/water

Suitable mixtures of the wash solvents may be as follows: heptane/ xylene in the ratio of from 0:90 to 100:10, suitably, of from 40:50 to 60:50, preferably of about 50:50.

Eluting the naphthenic acid-metal ion complexes may be achieved with a suitable eluant selected from the group consisting of C₆-aryl, straight chain or branched C₁-C₆ alkyl alcohol or C₆-aryl alcohol, ammonia, and straight-chain or branched C₁-C₆ alkylamine and mixtures thereof.

The term C₁-C₁₀ includes the groups methyl, ethyl, propyl, butyl, pentyl, hexyl, heptyl, octyl, nonyl, and decyl.

The C₁-C₆ alkylamine, includes primary, secondary and/or tertiary amines. The C₁-C₆ alkyl alcohol includes primary, secondary and/or tertiary alkyl alcohols. C₆-aryl alcohol includes phenol.

Preferably, the straight chain or branched C₁-C₆ alkyl alcohol may be methanol, ethanol, and/or isopropanol. Preferably, the C₁-C₁₀ alkane is *n*-heptane.

The straight chain or branched C₁-C₆ alkyl alcohol may be formulated as an aqueous solution or may be used. The straight chain or branched C₁-C₆ alkyl amine and/or ammonia may be formulated as an aqueous solution or as a solution of ammonia in a straight chain or branched C₁-C₆ alkyl alcohol as alcoholic ammonia or amine. For example, methanolic ammonia.

Suitably, the proportion of C₁-C₆ alkyl alcohol to ammonia or amine may be in the ratio of from 95:5 to 80:20.

Following, the elution of the complexed naphthenic acid-ammonia or amine co-ordinated transition metal ion, the eluate may be concentrated by evaporation of the elution solvents. The concentration of the same may be achieved by evaporation under an inert gas atmosphere (e.g. nitrogen or other inert gases like helium), lyophilisation or any other generally suitable means.

Quantitation of the concentration of naphthenic acid-metal ion complexes present in the eluate may be achieved by measuring the absorbance of complexed naphthenic acid-ammonia or amine co-ordinated transition metal ions present in the sample and correlating this absorbance from a calibration graph. The absorbance can be correlated to the concentration of the complexes present by using known standards as controls.

The formation of the complex yields an ion complex with a characteristic UltraViolet radiation (UV) absorption signal by virtue of the co-ordinated transition metal ion present in the complex. Quantitation of the amount of naphthenic acid in a sample may therefore be achieved using an assessment of the UV absorption of a sample by spectrophotometry techniques compared to known reference samples containing defined amounts of naphthenic acid, including samples where naphthenic acid is absent. Suitably, UV absorption measurement may be made at 215 to 225nm, preferably at 218 to 222nm.

Quantitation could also take place using means to detect the formation of radioactive complexes or complexes that give rise to fluorescent products.

The present invention therefore provides a newly developed screening method for isolation of and fingerprinting of a diagnostic family of high molecular weight naphthenic acids implicated in naphthenate soap material formation in oil production plants and in refinery facilities.

In a preferred embodiment of the invention, a method for the quantitation of naphthenic acid content in a hydrocarbon sample may comprise the steps of :
(1) Separating and purifying high molecular weight naphthenic acids from crude oil samples by e.g. dialysis
   (i) using a dialysis membrane sack in the separation of the high molecular weight naphthenic acids fraction from raw crude oil samples, petroleum fractions and deposits containing naphthenate soap (solids, soap-emulsions and sludges); and
   (ii) optionally using a metal mesh basket of specific dimensions may be used to protect the dialysis membrane sacks from bursting during distillative reflux dialysis extraction of test samples, with a basket holder for multiple samples to be run;
(2) Selecting a transition metal from the transition metal series;
(3) Preparing of a macro reticular resin in a transition metal form with a ligand as described above and forming resin as a column;
(4) Applying the sample to the column;
(5) Washing the column, and subsequent washing to remove excess uncomplexed sample with a heptane/xylene wash, or mixtures thereof, Isopropanol-water mixtures; and finally eluting the complexed naphthenic acid-co-ordinated ammonia/amine transition metal ion with an alcoholic amine solution;
(6) Identifying the ultraviolet (UV) absorption lambda max wavelength of metal co-ordinated naphthenic acid complexes from samples under test, with reference to known copper naphthenates (pure standards) and actual field naphthenate deposits acid fraction extracts, converted to the metal co-ordinated ammonium / amine forms, by elution through the macroreticular resin column;
(7) Identifying the optimum and maximum absorbance values required for metal co-ordinated naphthenic acid complexes, from samples under test, such that the absorbance remains within an acceptable lambda max range for the calibration graph;
(8) Identifying that the dilution of high absorbance's obtained from the metal co-ordinated naphthenic acid complexes meet with the maximum absorbance value recommended and that this dilution is linear;
   (i) Establishing a calibration procedure using high molecular weight naphthenic acids extracted from actual samples of naphthenate soap deposits/sludges recovered from actual oilfield production and refinery processes;
(9) Identifying the minimum volume of coloured, metal co-ordinated naphthenic acid complexes required for UV analysis and for UV fingerprint testing and identification of the minimum detectable naphthenic acids amount (Limit of Detection, LOD) in test solution, which is 1.25 mg in this method;
(10) Quantifying the amount of high molecular weight naphthenic acids (HMWA) obtained in the dialysis residue of test samples (e.g. crude oils, sludges) relative to known HMWA fractions extracted from actual oilfield deposit samples;
(11) Normalisation the %wt HMWA fraction in the dialysis residue of test samples back to the original crude oil; and
(12) Normalising the %wt HMWA fraction of stabilised crude oil samples with previously unknown %wt HMWA fraction / problematic soaps quality to the known problematic crude oils.

The UV fingerprinting method of crude oils allows mg content of HMWA to be determined from the calibration graph and this can then be expressed as the %wt HMWA fraction of each oil under test. This is used to screen oils by comparing them versus blank oils that do not form naphthenates in the oilfield and by comparing the results to crude oils known for having soap problems during production, including oils that give calcium naphthenate deposition.

Preferably the sample for analysis may be a crude oil sample, for example impure crude oil from exploration/appraisal wells, drill stem tests, test separator samples, downhole high pressure live samples (e.g. Schlumberger/Expro SPS/MDT/RCI type samples) or refinery charge to the heat exchangers. The sample could be crude oil residues recovered from oil refinery operations/plants. The sample could be crude oil or naphthenate deposits found in upstream petroleum processes or from refinery operations.

In the separation procedure, where dialysis is used, it may be preferable to use a basket or a holder for the dialysis membrane, such as a metal basket. The metal basket may be constructed of stainless steel which will not be oxidized or react with Low Molecular Weight Naphthenic (LMWA) acids.

Preferably, transition metal is chosen from a water soluble salt of Copper, Nickel or Molybdenum.

In the preparation of the co-ordinated transition metal ion, preferably the nitrogen containing ligand is chosen from ammonia, ethylamine and ethylene diamine.

After loading the column and washing of excess unbound transition metal ion and/or unreacted impurities from the sample, the elution solvent sequence can be chosen from a straight chain or branched C₁-C₁₀ alkane, C₁-C₁₀ alkane - C₆ aromatic solvent mix, aqueous C₁-C₆ alcohol, neat C₁-C₆ alcohol, and C₁-C₆ alcoholic ammonia / C₁-C₆ amine.

The neat alcohol may be methanol. The alkane may be chosen from C5, C6 or C7 alkanes or mixtures thereof, e.g., pentane, hexane and/or heptane. In the alcohol/amine mixture, the alcohol may be ethanol, methanol or isopropanol or mixtures thereof, but preferably methanol.

In a laboratory scale version of the method, the resin may be prepared as a column, for example in a burette. The burette may have a physical resin bed dimension of 35 cm x 10mm, with reservoir height of 15 cm. The resin may be Amberlyst A15®. In such embodiments, the sample dialysis weight may range between 1 to 8 g and the dialysis time may range between four to twelve hours. The preferred sample weight may be 6 grams. The preferred dialysis time may suitably be 6 hours.

Where dialysis is used in the separation of the sample prior to loading onto the resin, the dialysis reflux-extraction solvent may be pentane, hexane or heptane with hexane being preferred.

In a laboratory scaled version of the method, the charge of the dialysis residue on the burette sized column may range from 75 -250 mg, with a preferred range 75 - 150 mg. In such embodiments, the elution rate may range from 1.5 - 3 ml/min, with a preferred rate of 2 ml/min. In such embodiments, the volume of eluates to be used may be standardised at 100 ml volume. Glacial acetic acid or formic acid can be used in sample loadings to a maximum of 20 micro litres on the burette sized columns.

After elution of the naphthenic acid complexes from the column, the alcohol or alcohol-ammonia solutions may be gently evaporated off to dryness under a nitrogen blanket.

The residues obtained from the evaporation of the alcohol or alcoholic ammonia may be collected and combined in a suitable amount of alcoholic ammonia solution for UV analysis starting at 3 millilitres and concentrated by evaporation to a residue.

Standard naphthenic acids may be obtained as follows from source hydrocarbons. Crude naphthenate soaps may be isolated from crude oil by a clean up step then followed by a dialysis extraction procedure. Warm heptane and ambient temperature xylene may be used in the clean up step. After the clean-up step, the crude naphthenate soap may then be treated to remove oil and residues/asphaltenes. The DR after dialysis extraction may be treated with warm (60°C) xylene-glacial acetic acid (1:1), extract solution, evaporated to dryness then re-dialysed twice to give the purified field naphthenic acids on evaporation to a residue.

The analysis of the amount of naphthenic acid in a sample or a standard may be carried out by measuring the UV absorption of a sample, wherein the lambda max UV absorption is set at 218-222 nm +/- 2 nm. Preferably, the maximum absorbance at lambda max range of 218-222nm is set at 2.5 +/- 0.5nm. The HMWA absorbances across the UV range may be measured on a UV-computer link and results normalized to those of known crudes, enabling prediction of problematic soaps formation.

The oil analysis method describe in the present application involves a novel combination of analytical techniques, which concentrate up the high molecular weight acids fraction of any crude oil sample. While concentrating on actual crude oils samples with a view to using this analysis technique to screen them for their acid fraction contents, it is equally possible to use this method on samples of sludge, solid deposits, treatment chemicals, drilling muds, distilled crude oil fractions, oil products and so on. These isolated acids with other high molecular weight components are then complexed on to a specific type of resin column for later elution and ultraviolet (UV) spectrophotometric analysis. Contaminants are washed off the column first, leaving behind the organic acids of interest. Results show a gradation of % complexed acids versus good and bad oils relative to probability of soap formation resulting in oil production and/or oil refining problems.

This technique holds great potential as an oil screening tool for new oilfields and even can be used on current production operations for troubleshooting present problems, i.e. with compartmentalised oil reservoirs, on a well-by-well basis, you can determine the wells that give fluids that will generate soaps and those that do not, allowing a map to be built up of fluids produced versus potential soap generation. The method procedure is relatively rapid, simple to learn and run, very accurate when compared to the other oil screening techniques currently available and only a small quantity of the test oil sample is required for this test method.

Using such a method to rank oils with respect to their risk of causing soap problems, in upstream oilfield production systems and in downstream crude refining plants, is the basis of this patent application.

The main justification for this new oil analysis screening method is the determination of the % weight high molecular weight naphthenic (%HMW) acids fraction of crude oils, leading to a classification fingerprint index. This index will assist in evaluating crude oils from new oilfield developments because once analysed, they can be classified with respect to their degree of risk for naphthenate soap formation, relative to the % HMW naphthenic acids. This prediction of problematic naphthenate soap formation is becoming critically important for new oilfield developments and their production facilities process design. Operating companies need to know up front if a new oil will be a naphthenate soap former or not, as the associated flow assurance issues can be very severe and will heavily influence the facilities design [5]. In addition, such operating companies can look at their overall oilfield development plan and decide upon the various alternative measures they could take in controlling any naphthenate related production problems, from wells to the topsides export oil system. These options would include dilution of problem oils with oils that will not form naphthenates, dilution of the produced waters to negate naphthenate formation, injection of treatment chemicals for naphthenate soap control, employing up front high pressure separators and so on [2 to 5 & 8]. This oil screening tool for naphthenates potential will have a big impact on the decisions made by various oilfield operating companies in the future.

The present invention has taken the analysis of heavy naphthenic acid soap precursors a step further by using a combination of exhaustive distillative-reflux and dialysis separation techniques, giving a concentration of high molecular weight naphthenic acids and then further reaction of the naphthenic acids by trans-metallation to metal ligand co-ordinated-ammonium naphthenates, on a macroreticular resin column. This leads to highly UV active transition metal-complexed compounds, which can be detected, with a suitable scanning UV spectrophotometer, down to the 1.25 mg level, in actual test solutions.

The bulk concentration of polar compounds based upon molecular weight can be performed by a separation technique known as dialysis, which is here paired with a second separation phase using macroreticular resin. Normal column chromatography, using alumina and silica gel, separates materials by polarity strength, which is related to molecular weight. Separation by normal column work is not bulk separation in groups but fine separation due to material polarities. Preparatory Thin layer Chromatography (PTLC) gives the same types of separations at narrow MW ranges, but it is difficult to use TLC on crude oil samples, as alumina will react with the naphthenic acids.

Dialysis is normally used in the well-known medical fields in blood purification re kidney dialysis. References are available in the food industry for pumped dialysis in non-organic electrolytes [27]. This type of dialysis is not the closed sack distillative reflux dialysis but pumped dialysis or direct current dialysis at ambient conditions.

References also exist for the macroreticular resins but mainly used in the environmental field. Silver is chemically and physically bonded to a macroporous resin (macroreticular), to enable it to scavenge out biocides from effluent waters [28]. No reference has been found, though, for the use of these macroreticular resins in their amino/ammonia-metal ligand form, in separation of naphthenic acids from crude oils, by column complexation and trans metallation. Types of anion exchange resins have been used to separate naphthenic acids [10, 29] but not in the area of direct metal ammonia-coordinated complexations of naphthenic acids from crude oils. This is one reason why this methodology for patent exists because it has not been tried before.

Based on the synopsis of the naphthenate soap problems discussed previously there is a need for a less expensive, less time-consuming, more accurate and commercially more cost-effective analytical technique for screening oil samples for naphthenate formation potential. This development work has shown advances in the application of combined analytical techniques, including separation of a specific molecular weight range of organic acids from crude oil and complexation of that acid fraction, to give a method, which can be used in actual onsite oilfield laboratories, as well as in a central land-based laboratory.

Preferred features for the second and subsequent aspects of the invention are as for the first aspect *mutatis mutandis.*

The invention will now be further described by way of reference to the following Examples and Figures which are provided for the purposes of illustration only and are not to be construed as being limiting on the invention. Reference is made to a number of Figures in which:
FIGURE 1 shows Compilation of Properties of Crude Oils versus %HMW acids; Note 1. HMWA back normalized to the original oil; Note 2 NSEA-B2 Normalized accounts for % BSW, % volatiles & % insoluble crystals in the DR, compared to NSEA-B sample.
FIGURE 2 shows Graphical Representation of Table 4 Data; Calibration Graph of Purified Naphthenic acids, Absorbance of Naphthenic acid complexes from resin column v mg Naphthenic Acids used in Final test solution; Note 1. The graph shows a gradient of 1 Absorbance unit = 5.4 +/- 0.2 mg HMW naphthenic acids in final test solution

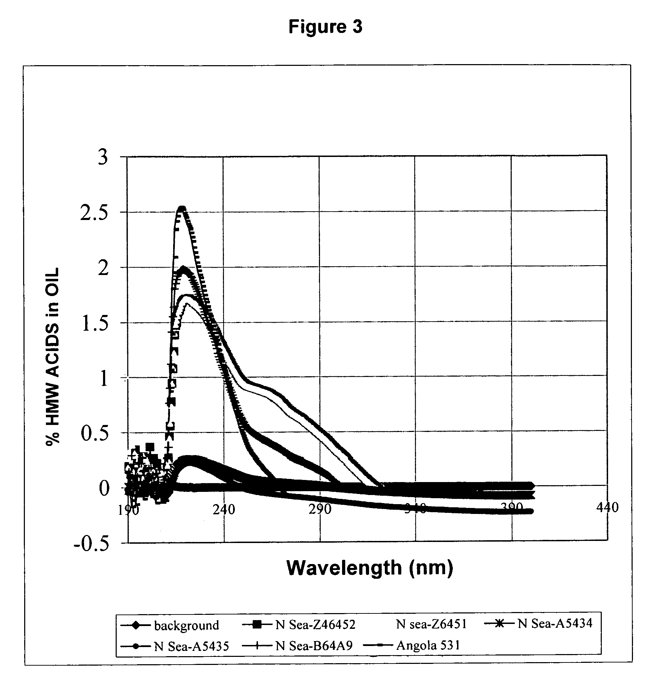

FIGURE 3 shows Variation of the % High Molecular Weight Acids in Selected Crude Oils versus Wavelength in UV Region; Note 1. Graph of curves, computer normalized for baseline correction and relative to sample weight & % DR; Note 2. N Sea-B curve is not adjusted for crystals found in the DR nor % BSW or % Involatiles, A corrected value for 20% crystals shows a 1.6% HMWA in oil. Graph shows N Sea-Z has an elevated concentration of HMWA in the DR; Note 3. N Sea-B totally normalized: BSW, % Involatiles, % crystals gives 0.6% HMWA acids in crude oil

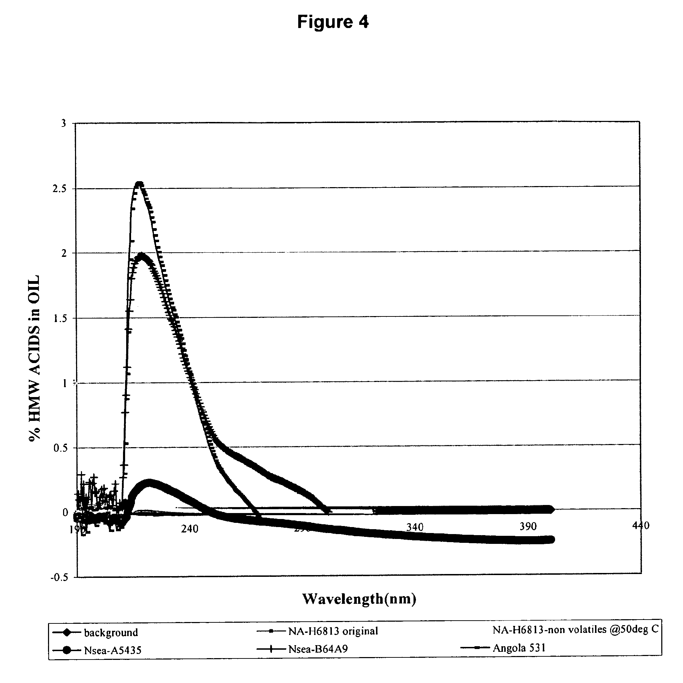

FIGURE 4 shows Variation of the % High Molecular Weight Acids in Crude Oils comparing N Sea-A, N Sea-B, Angola and NA-H; Note 1. NA-H, yellow & red curves, (0.05 TAN), a well in a new oilfield shows < 0.1 % HMWA in the crude oil

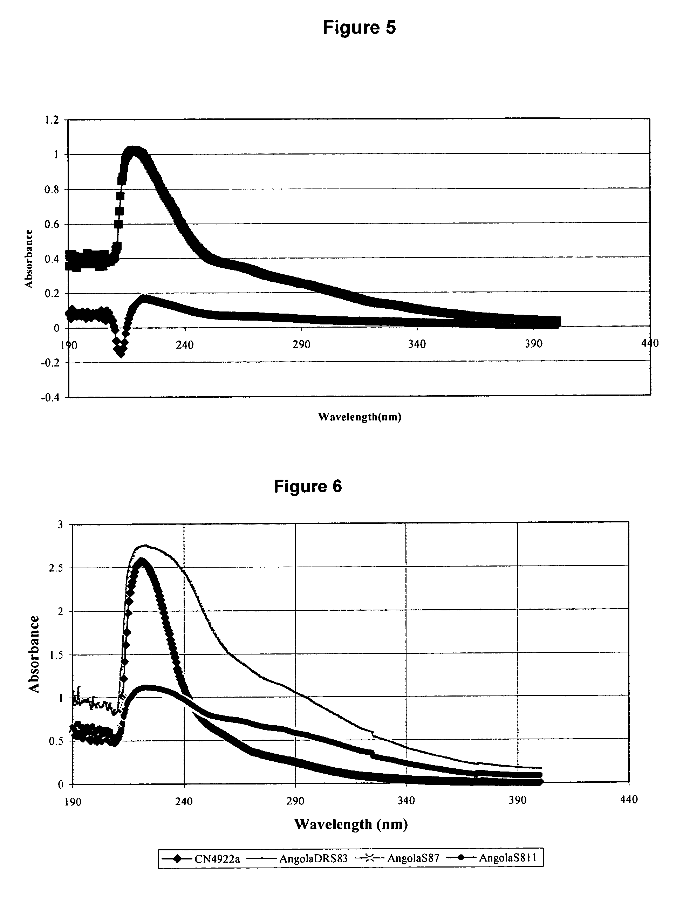

FIGURE 5 shows Graph showing the Low Interference Absorbance of Asphaltenes charged at 45 mg on column, compared to complexed Naphthenic acids from West Africa-K Oil HMW Naphthenic acids; Note 1. Asphaltenes at 50% level of contamination in a normal DR charge do not adversely interfere with the Cu complexed naphthenic acids. The 45 mg asphaltenes gave < +2% error to the Naphthenic acid recovery.
FIGURE 6 shows Comparison of Complexed Angola Naphthenic acids from the Crude DR v Standard Fluka Cu Naphthenate in methanol-NH3 solution; Note 1. Graph above depicts similarities between standard Fluka Copper Naphthenate in ammoniacal form compared to Angola Naphthenic acids eluted, complexed in the cuprammonium ligand state .The Angola Naphthenic acids form a broad spectrum of high molecular weight acids.

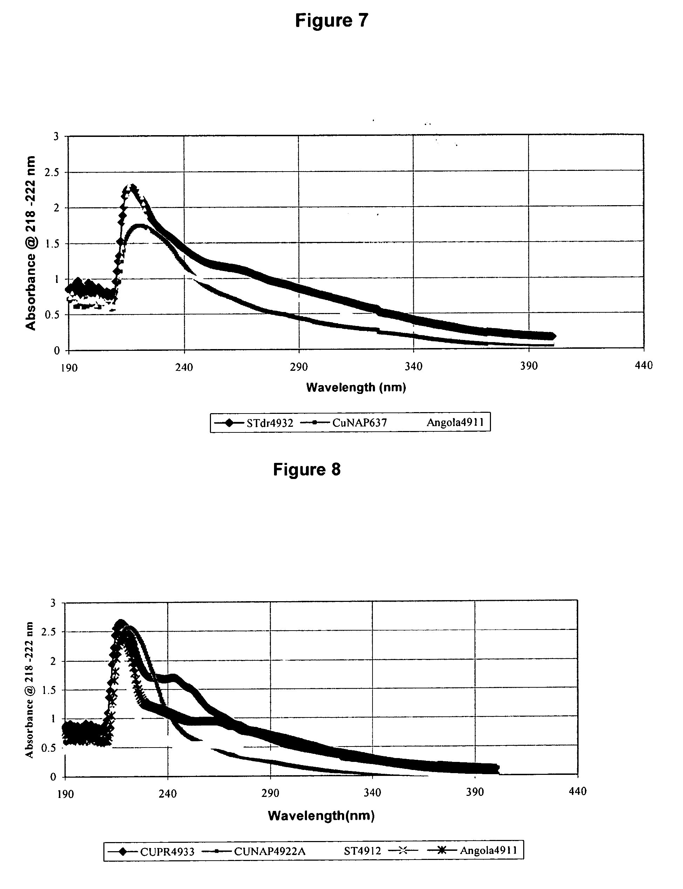

FIGURE 7 shows Comparison of Angola & Australia-S DR Naphthenic acids v Fluka standard Copper Naphthenate in acid form-All eluted as Cuprammonium Naphthenates ex Cu MRR column; Note 1. Australia-S, Angola and the standard Fluka Copper Naphthenates eluted as cuprammonium naphthenate complexes from the column, show good similarities, matching the fingerprint patterns.
FIGURE 8 shows Graph showing comparison of Cuprinol & Standard Fluka Copper Naphthenates, Eluted with Ammonia ligands in Methanolic Ammonia vs Australia-S & Angola Cuprammonium Naphthenates from Cu-MRR column; Note 1. Cuprinol Copper Naphthenate, wood preservative residue, is compared with Fluka Copper Naphthenate eluted with Methanolic Ammonia from the Cu-MRR column and compared with Australia-S and Angola to complexed Cuprammonium Naphthenates from the column. Generally a good agreement in fingerprint patterns is observed.

### Example 1: Analysis of crude oil for naphthenic acid content

To validate and proof this new oil screening methodology, various crude oils were carefully chosen based upon physical data to represent both known oils with actual calcium naphthenate formation problems in the oilfield and oils with absolutely no known calcium naphthenate soap-forming problems (listed in Tables 1 and 2 and Figure 1). Initially, the crude oils under test were stabilised gravimetrically utilising nitrogen-purged heating to remove light ends (while not removing the heavy naphthenic acids that were being targeted in the present study) and the water content would be separated by the standard heated centrifuge method.

It was discovered that dialysis membranes can burst by rapid expansion due to volatiles loss from crude oils, hence the initial heating to remove light ends. A stainless steel metal cage was constructed to hold the dialysis sack containing the sample and locked in position such that the sack does not expand to bursting. The dimensions of the metal cage were chosen such that several metal cages containing samples can be stacked to fit the 1 litre dialysis reservoir. This then meant that approximately eight oil samples could be dialysed in one distillative reflux dialysis experiment. Samples were initially stabilized by gently heating up a known weight of sample under a nitrogen blanket to prevent oxidation. Each sample undergoes a heat - cool procedure to constant weight. The weight loss of volatiles is recorded and used in a correction procedure. The free water is removed by pipette or by a 60 seconds centrifuge spin (heated). The amount of removed free water (weight loss) is also used in a correction procedure when required.

The stabilised oils were then extracted by use an exhaustive distillative-reflux membrane dialysis apparatus and the sack retained non-dialyzable high molecular weight acids (HMWA), with a MW range of approximately 600 amu and above (including the naphthenate precursor ARN-type acids), which included any naphthenate soaps present. These would then be gravimetrically collected by removal of the extraction solvent by heating at 70 deg C, under a Nitrogen blanket. (DR weight is recorded and converted to % DR), minus the sack weight), and termed as the dialysis residue (DR). The samples include solids, soap-emulsions and sludges. The DR material containing these acids of interest stays isolated inside the sack.

The solvent dialysate (DS) containing low molecular weight compounds including low molecular weight (LMW) naphthenic acids was not used further in this stage of this method. The DR's were dissolved and volumetrically prepared in normal organic solvents and eluted through a specially prepared copper ammonium macroreticular resin column using a series of chosen solvents (by experiments). This combination of solvents removes, residual oil and asphaltenes/resins/waxes, leaving the high molecular weight naphthenic acids of interest, complexed (bound) on the column.

Copper is chosen here in the example but transition metals give coloured complexes from their oxidized states when they accept a ligand. Copper, Nickel and Molybdenum are safe to work with. Chromium is toxic so not used here. The transition metal is chemically bonded on to the macroreticular resin and then prepared to accept an amine/ammonium ligand. The macroreticular resin column is first converted to the transition metal form and co-ordinated with ammonia or amines from above. The ammonia/amines attach to the transition metal and rapidly impart a characteristic blue colour to the co-ordinated metal still attached to the resin. This complex now reacts with naphthenic acids to give coloured complexes based on the amount of naphthenic acids attached to the metal. For example the cuprammonium ion does not give a UV spectra, but once complexed with naphthenic acids, a UV absorption is observed in the copper - carbonyl UV absorption area.

Xylene and heptane are used to clean up heavy oil residues if these are present in the DR charged to the column. Xylene and toluene were found to be good solvents for these residues. IPA-water solvent clean up after the xylene wash, affords a swelling of the column to expand the pores of the resin to remove the residual xylene and thus the residual heavy residues if present. The sequential solvent washing was identified after lengthy trials and it removes contaminants while leaving the acids of interest on the column. These coordinated metal complexed naphthenic acids would then be collected in two experimentally chosen elution solvents and carefully evaporated under nitrogen purge, slowly to dryness. The residues would then be combined, re-dissolved in an ammoniacal solution for spectrophotometric analysis. Results examples for some oils are shown in Tables 1 to 3 and in Figures 1, 3 and 4. UV absorbance values are compared to a standard calibration graph constructed in the same manner, using doubly purified high molecular weight naphthenic acids derived from the purified tan coloured naphthenic acid soaps, extracted from an actual calcium naphthenate scale deposit from an Angolan oilfield (see Table 4 and Figure 2). Asphaltene solids recovered from a real production system in Thailand were found not to interfere with the UV signal diagnostic for the naphthenic acid fractions isolated from the oil samples. Less than 2% relative increase in HMWA is noted for the UV signal observed at 50% asphaltenes contamination of the DR (see Figure 5). Other components of the DR held on the column e.g. clay, but inorganics are not mobile and will not interfere with the test. Other possible interferents like corrosion inhibitors & wax inhibitors have been tested by the method and found not to interfere with the UV fingerprint of the acids of interest.

The UV spectra were also compared to those of two commercially available (wood preservatives) [30, 31] naphthenate residues (petrochemicals derived from crude oil), converted to the metal complexed ammoniacal forms. UV fingerprinting results of oils compared to these standard naphthenates and are shown in Figures 6, 7 and 8

This method is repeatable and reproducible across operators and columns to within 2.5% relative and at approximately 50% threshold loading for the columns lengths and volumes being used at present. Limit of detection at present using burette-sized columns is estimated at 1.25 mg in actual test solutions, with present UV spectrophotometer. The method and results afford a good correlation table of % high molecular weight acids (HMWA) in oil, versus degree of critical naphthenate soap formation, see Table 1. The weight of the dialysis residue gives a weight % of inherent fouling material. The DR will contain the above-mentioned HMW materials and others comprising of inorganics, clay, sand, dirt, scale, corrosion byproducts etc. This includes naphthenate soaps, HMWA, asphaltenes, resins and inorganic materials.

Normalization of the results for the UV absorbance (see Figure 4) is performed from a spreadsheet using the factor of 1 Abs unit = 5.44 mg of HMWA in the DR. The UV absorbance used here is corrected for baseline absorbance. The mg HMWA obtained for the weight of DR is converted to total HMWA in the DR, by multiplying by the UV dilution factor if used, and then this HMWA value is now corrected to % HMWA in the DR. This value of % HMWA in the DR is back normalized to the % in the original stabilized oil by multiplying by the % DR. Further comparison normalization within a series of known problematic or non-problematic crudes is performed by using the formula for % HMWA in original oil and plotting these on a graph, superimposing several known samples for comparison. These oils used for comparison include oilfields operating for many years without any known calcium naphthenate soap problems, namely the blank crude oil cases but also crude oils from oilfields with actual calcium naphthenate deposition problems and other oilfields with sodium and potassium ultra-stable soap-stabilised emulsion problems. A few of the cuprammonium co-ordinated naphthenates eluted from the columns were evaporated to dryness and the residues were treated with HAC/Xylene to release the naphthenic acids. The naphthenic acids were analysed by electrospray ionisation - mass spectrometry (ESI-MS), which showed the HMWA including the ARN acid patterns as proof that HMWA were being complexed on the column. The embedded file on the next page gives the results of this comparison.

The present invention herein is provided, a method, justly relating to the requirements of the Energy sector and the need for a rapid, reliable, accurate, non-costly field and laboratory method for determination of the relative % of high molecular acids in crude oils, crude oil fractions, sludges and naphthenate soap deposits.

The method covers such organic acids and salts of these acids as can be extracted from raw 'as received' petroleum crude oil liquid samples (either dry or water containing), as well as acids extracted from oilfield production system deposits, scales, sludges and emulsions, including acids extracted from petroleum fractions of crude oils. This quantitative method isolates these acids to such a degree that their concentration can be determined in units of milligrams (mg) acid per litre of oil or sample (mg/l) and the results can be expressed as % weight (% wt) acid fraction of oil, henceforth known as % High Molecular Weight Acid (% HMWA) fraction (see Table 1). This specific group of naphthenic acids that this method targets are known to be in part responsible for the formation of problematic calcium naphthenate soap solids deposition and/or in the formation of sodium or potassium soap emulsion problems in many oilfield production facilities worldwide.

### References

1. Goldzal et al 3rd Intl Conf on Petroleum Phase Behaviour and Fouling, (2002), New Orleans, USA
2. Rousseau et al "Calcium Carbonate and Naphthenate Mixed Scale in Deep Offshore Fields", SPE 68307 (2001).
3. Vinstad et al "Fighting Naphthenate Deposition at the Heidrun Field", (2003). SPE Oilfield Scale Symposium, SPE 80375
4. Roden, J, "Calcium Naphthenate Control on the Blake Field", proceedings from Flow Assurance Europe (IQPC 18th - 19th November 2003, London) (2003).
5. M.S Turner & P.C Smith (2005), Controls On Soap Scale Formation, Including Naphthenate Soaps - Drivers And Mitigation, SPE Oilfield Scale Symposium, SPE 94339, Aberdeen, UK
6. Sorbie et al 'Naphthenate Formation In Oil Production: General Theories And Field Observations', RSC; Chemistry in the Oil Industry IX, Manchester, UK, 31 October - 2 November 2005
7. Baugh et al SPE 93011, SPE Intl Symposium on Oilfield Scale, Aberdeen, UK, 1-12 May 2005
8. Gallup, D. L.: "Ca-Naphthenates and Metallic Soap Deposits: Formation and Mitigation", IQPC - Flow Assurance - A Holistic Approach, December 2004, Kuala Lumpur (2004).
9. Haynes, D. (2002) Opportunities in Processing High TAN Crude. Presented at Hydrocarbon Asia (September 2002).
10. Medias et al, 5th SPE Oilfield Scale Symposium, SPE 8404, (2003) Aberdeen, UK.
11. Acevedo et al Energy Fuels 1999, 13, 333-335.
12. Lochte, H.L., & Littman, E.R., The Petroleum Acids and Bases., Chemical Publishing Co., Inc, NY 1955
13. Seifert, W.K & Howells, W.G. Anal Chem. 1969, 41, 554-568
14. Seifert, W.K & Teeter, R.M,. Anal Chem. 1969, 41, 786-795
15. Seifert, W. K, & Teeter R.M. Anal Chem. 1970, 42, 180-189
16. Oil Plus internal fluid characterisation reports (not published).
17. Naphthenic Acid Corrosion in Crude Distillation Units, Material Performance, 28, 1988, 32-43
18. Goldzal et al, SPE 74661, SPE Intl Symposium on Oilfield Scale, Aberdeen, UK, 2002
19. Brandl, O, PhD Thesis, Norwegian Inst of Sci and Tech, May 2005
20. Derungs, W.A. Corrosion, 1956, 12, 41-46
21. Babian-Kibala et al Amer. Chem. Soc., 1998,3, 106-110
22. Roussis et al (ExxonMobil) WO 02/48698 - 20 June 2003
23. Green et al High Resoluti. Chromatgr. 1994, 17, 427-438
24. Havre et al J Dispersion Sci. Techol., 2002.
25. McCarthy, R. & Duthie, A.J. J. Lipid. Res. 1962, 3, 117-119
26. Havre, E. T, Colloid Polym. Sci. (2004), 282, 270-279
27. Membrane Dialysis: determination of sugars in Dairy Products, Metrohm UK, Laboratory Talk, 2 July 2004
28. Costin, C. R., Rohm & Haas Company, PA, US 4076622, 1975
29. Tomczyk et al Prepr.-Am, Chem, Soc., Div, Pet, Chem., 1998, 3, 125, 123-125
30. Brient J.A. Prepr. -J.Amer. Chem. Soc., Div. Pet. Chem. 1998, 3, 131-133

**Table 1**

| Test Oil Identification | DR % weight | Ca Naphthenate Risk | TAN (mg KOH / g oil) | API | Weight DR used (mg) | Corrected Absorbance | Dilution | mg HMW Acids detected | % HMW acids in DR | % HMW acids in oil |
|---|---|---|---|---|---|---|---|---|---|---|
| Norway | 2.6 | yes | 2.7 | 21 | 98 | 1.6 | 4.5 | 39 | 39.5 | 1.26 |
| Thailand | 4.1 | no | 0.09 | 35 | 100 | | 1 | 7.5 | 7.5 | 0.03 |
| Australia-S | 3.6 | PY | 1.9 | 19 | 151 | 2.2 | 5 | 59 | 41.3 | 1.46 |
| Angola1 | 8.15 | yes | 2.7 | 18 | 113 | 1.16 | 4 | 39.3 | 26.2 | 2.5 |
| Indonesia | 3.94 | no | 0.55 | 40 | 98 | 1.71 | 1 | 9.2 | 9.44 | 0.37 |
| N Sea-A | 1.89 | no | 1.2 | 20 | 65 | 1.5 | 1 | 8 | 12.6 | 0.36 |
| Angola2 | 8.15 | yes | 2.7 | 22 | 88 | 1.3 | 4 | 28 | 31.9 | 2.5 |
| Angola3 | 8.15 | yes | 2.7 | 22 | 113 | 1.48 | 3.5 | 29.2 | 26 | 2.45 |
| N Sea-Z | 18.5 | PY | 0.12 | 31 | 199 | 1.16 | 3 | 18.6 | 9.35 | 1.74 |
| N Sea-B | 1.17 | yes | 0.09 | 32 | 53 | 1.17 | 4 | 25.3 | 47 | 0.6 |
| N Sea-W | 6.4 | yes | 2.6 | 22 | 145 | 1.8 | 5 | 51 | 35.5 | 2.27 |
| West Africa-K | 7.1 | no | 6.0 | 17-20 | 59 | 0.77 | 1 | 4.2 | 7.1 | 0.5 |
| West Africa-B | 13.1 | PY | 4.35 | 21 | 223 | 1.8 | | 29.2 | 13.1 | 1.7 |
| N Africa-H | 0.41 | PN | 0.05 | 30 | 22 | 0.02 | | 1.1 | 5.2 | 0.02 |
| N Sea-B2 | 3.5 | yes | 0.09 | 32 | 53 | 1.17 | 4 | 25.3 | 47 | 1.74 |

| | | | | | | | | | | |
|---|---|---|---|---|---|---|---|---|---|---|
| Notes: HMW = High Molecular Weight Acids DR = Dialysis Residue TAN = Total Acid Value in units of mg KOH / g oil Corrected Absorbance = Total absorbance - Baseline Absorbance Ca Naphthenate Risk: in column 3. PY= Predict Yes, PN =Predict No. NSEA-B results uncorrected for % volatiles, and % BSW, note difference in % DR used between NSEA-B and B2 | | | | | | | | | | |

**Table 2**

| Correlation Table of Three Important Properties of Crude Oils Analysed including % High Molecular Weight Acids in the oil. | | | |
|---|---|---|---|
| Field ID code | % DR | TAN | %HMW acids in Original oil |
| North Sea-Z | 18.6 | 0.12 | 1.74 |
| Angola-2 | 8.45 | 2.7 | 2.5 |
| Angola-3 | 8.45 | 2.7 | 2.45 |
| Angola-1 | 8.45 | 2.7 | 2.5 |
| Australia-S | 3.6 | 1.9 | 1.46 |
| Norway | 3.2 | 2.7 | 1.26 |
| Indonesia | 3.94 | 0.55 | 0.37 |
| North Sea-A | 1.90 | 1.2 | 0.36 |
| Thailand | 4.1 | 0.09 | 0.03 |
| North Sea-B | 3.51 | 0.09 | 1.64 |
| North Sea-W | 6.4 | 2.6 | 2.27 |
| West Africa-K | 7.12 | 6 | 0.503 |
| West Africa-B | 13.1 | 4.35 | 1.708 |
| N sea-B2 corrected for BSW, %Invols | 1.2 | 0.09 | 0.6 |
| N Africa-H | 0.41 | 0.05 | 0.02 |

| | | | |
|---|---|---|---|
| Note 1: Results for N Sea-B corrected for ~ 20% w crystals found in the DR. Note 2. Values obtained for mg HMWA in DR are interpolated from the computer trended graph. | | | |

**Table 3**

| Ranking correlation of %HMW Acids of Crude Oils Analysed | |
|---|---|
| Crude oil | %HMW acids in Original oil |
| Angola-2 | 2.6 |
| Angola-1 | 2.5 |
| Angola-3 | 2.45 |
| N Sea-W | 2.27 |
| N Sea-Z | 1.74 |
| West Africa-B | 1.71 |
| N Sea-B (2) | 1.64 |
| Australia-S | 1.46 |
| Norway | 1,26 |
| N Sea-B (3) | 0.6 |
| Indonesia | 0.37 |
| N Sea-A | 0.36 |
| West Africa-K | 0.50 |
| Thailand | 0.03 |
| NA-H4 | 0.02 |

| | |
|---|---|
| Note 2: Results for N Sea-B corrected for ~ 20% w crystals found in the DR; Note 3: N Sea-B (3) normalized for % BSW, % involatiles & % crystals in DR; Angola 1-3 results are in triplicate. | |

**Table 4 - Calibration Data for Naphthenic acids Extracted from an Actual Angolan Oilfield Naphthenate Deposit, Complexed on the Column**

| Naphthenic acid (mg) | Total Absorbance A | Corrected Absorbance A |
|---|---|---|
| 13.5 | 3.403 | 2.55 |
| 10 | 2.825 | 2.03 |
| 5 | 1.48 | 1.10 |
| 2.5 | 0.67 | 0.37 |
| 1.25 | 0.63 | 0.35 |

| | | |
|---|---|---|
| Note 1. Corrected Absorbance A is from the linear regression applied to the data. | | |

**Table 5 - Summary of Method Analysis Results**

| | | | | | | | | | | | |
|---|---|---|---|---|---|---|---|---|---|---|---|
| Crude oil | N SE-A | N SEA-W | NORWAY | Australia-S | ANGOL A | N SEA-B | N SEA-Z | Indonesia-S | W Africa-K | W Africa-B | NA-H |
| %BSW | 0.100 | 0.100 | 0.100 | 0.100 | 0.100 | 49.000 | 0.100 | 0.100 | 20.000 | 0.100 | 0.100 |
| %Dry/Stabilized crude | 99.900 | 99.900 | 99.900 | 99.900 | 99.900 | 51.000 | 99.900 | 99.900 | 80.000 | 99.900 | 99.900 |
| TAN mg KOH/g | 1.200 | 2.600 | 2.700 | 1.900 | 2.700 | 0.090 | 0.120 | 0.550 | 6 | 4.35 | 0.050 |
| API | | | | | 18.000 | | | | | | |
| | | | | | | | | | | | |
| Weight sample | 7.500 | 5.671 | 8.200 | 4.052 | 7.350 | 12.476 | 7.512 | 9.502 | 7.350 | 7.500 | 10.000 |
| Weight sample after heating @45C | 7.500 | 5.671 | 8.200 | 4.052 | 7.289 | 4.451 | 7.409 | 9.502 | 6.852 | 7.030 | 4.400 |
| % Residual | 99.900 | 100.00 | 99.900 | 99.900 | 99.071 | 33.195 | 98.530 | 99.900 | 74.580 | 93.640 | 43.956 |
| | | | | | | | | | | | |
| weight dry/residual crude taken for DR | 6.600 | 5.671 | 8.200 | 4.052 | 5.290 | 4.643 | 7.055 | 6.612 | 4.933 | 6.394 | 2.458 |
| DR-final volume used | 22.000 | 25.000 | 20.000 | 10.000 | 10.000 | 10.000 | 40.000 | 20.000 | 40.000 | 40.000 | 20.000 |
| Volume of DR solution taken for test | 7.500 | 10.000 | 7.500 | 10.000 | 10.000 | 10.000 | 15.000 | 7.500 | 5.000 | 10.000 | 5.000 |
| | | | | | | | | | | | |
| Residual Volume of DR solution | 14.500 | 15.000 | 12.500 | 10.000 | 0.000 | 0.000 | 25.000 | 12.500 | 35.000 | 30.000 | 15.000 |
| Weight (g) DR in residual volume | 0.124 | 0.218 | 0.164 | 0.144 | 0.435 | 0.000 | 0.830 | 0.163 | 0.412 | 0.669 | 0.017 |
| Corrected weight of DR | 0.188 | 0.363 | 0.262 | 0.144 | 0.435 | 0.163 | 1.328 | 0.261 | 0.471 | 0.892 | 0.023 |
| wt insolubles in DR | | | | | | 0.034 | | | | | |
| %DR | 2.848 | 6.407 | 3.197 | 3.550 | 8.147 | 3.511 | 18.547 | 3.940 | 7.119 | 13.063 | 0.405 |
| (1 A = 5.4 mg NA in final solution) | 5.400 | 5.400 | 5.400 | 5.400 | 5.400 | 5.400 | 5.400 | 5.400 | 5.400 | 5.400 | 5.400 |
| Baseline Abs A | 0.800 | 0.900 | 1.000 | 0.000 | 0.700 | 0.800 | 0.650 | 0.700 | 0.400 | 0.700 | 0.150 |
| Total abs A | 2.300 | 2.810 | 2.600 | 2.200 | 2.000 | 1.970 | 1.800 | 2.410 | 1.170 | 2.500 | 0.360 |
| Corrected Abs A | 1.500 | 1.910 | 1.600 | 2.200 | 1.300 | 1.170 | 1.150 | 1.710 | 0.770 | 1.800 | 0.210 |
| Dilution factor for UV | 1.000 | 5.000 | 4.500 | 5.000 | 4.000 | 4.000 | 3.000 | 1.000 | 1.000 | 3.000 | 1.000 |
| | | | | | | | | | | | |
| Weight mg of DR taken for test | 64.138 | 145.333 | 98.400 | 144.000 | 88.000 | 53.000 | 199.200 | 97.800 | 58.857 | 223.000 | 22.000 |
| | | | | | | | | | | | |
| Weight mg of HMW acids detected | 8.100 | 51.570 | 38.880 | 59.400 | 28.080 | 25.272 | 18.630 | 9.234 | 4.158 | 29.160 | 1.134 |
| | | | | | | | | | | | |
| % HMW acids in DR | 12.629 | 35.484 | 39.512 | 41.250 | 31.909 | 47.683 | 9.352 | 9.442 | 7.065 | 13.076 | 5.155 |
| | | | | | | | | | | | |
| %HMW acids in original oil | 0.360 | 2.273 | 1.263 | 1.464 | 2.600 | 1.674 | 1.735 | 0.372 | 0.503 | 1.708 | 0.021 |
| | | | | | | | | | | | |
| Yala: % DR | 4.100 | 4.100 | 4.100 | 4.100 | 4.100 | 4.100 | 4.100 | 4.100 | 4.100 | 4.100 | 4.100 |
| Ref Y: milligrams DR used | 100.000 | 100.000 | 100.000 | 100.000 | 100.000 | 100.000 | 100.000 | 100.000 | 100.000 | 100.000 | 100.000 |
| Ref Y: weight mg HMW acids detected | 7.500 | 7.500 | 7.500 | 7.500 | 7.500 | 7.500 | 7.500 | 7.500 | 7.500 | 7.500 | 7.500 |
| Ref Y: %HMWA in oil | 0.300 | | | | | | | | | | |

| | | | | | | | | | | | |
|---|---|---|---|---|---|---|---|---|---|---|---|
| Formula for UV computer data normalization = Normalized Absorbance Value= ((((Abs-Baseline)* UV Dilution)*5.44)*%DR)/mg DR used | | | | | | | | | | | |

## Claims

1. A method for the quantitation of the naphthenic acid content in a hydrocarbon composition, comprising
(i) separating naphthenic acid from the hydrocarbon composition;
(ii) applying the separated naphthenic acid to a macro reticular ion-exchange resin additionally comprising transition metal ions co-ordinated with an amine or ammonia in which the metal ion amine/ammonia co-ordinated complexes are bound to the surface of the resin;
(iii) washing the column;
(iv) eluting the naphthenic acid-metal ion complexes from the column; and
(v) quantitating the concentration of naphthenic acid-metal ion complexes present in the eluate from the column.

2. A method as claimed in claim 1, in which the hydrocarbon composition comprises crude oil.

3. A method as claimed in claim 1 or claim 2, in which the step (i) of separating naphthenic acid from the hydrocarbon composition is carried out by dialysis.

4. A method as claimed in any one of claims 1 to 3, in which the macro reticular ion-exchange resin comprises a transition metal ion selected from Copper (Cu), Silver (Ag), Nickel (Ni), Cobalt (Co), Iron (Fe), Manganese (Mn), Chromium (Cr), and Platinum (Pt).

5. A method as claimed in any one of claims 1 to 4, in which the amine is of general formula: 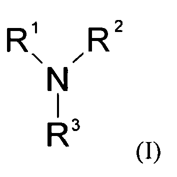 in which R¹, R² and R³ may each independently be Hydrogen (H), or a C₁-C₆ straight chain or branched alkyl group.

6. A method as claimed in claim 5, in which the amine is ethyl amine, ethylene diamine, morpholine, pyridine, cyclohexylamine or benzylamine.

7. A method as claimed in any preceding claim, in which washing of the column in step (iii) is carried out using xylene, toluene and/or heptane

8. A method as claimed in claim 7, in which the step (iii) is followed by a further wash using isopropanol-water mixture.

9. A method as claimed in preceding claim, in which the eluting the naphthenic acid-metal ion complexes in step (iv) is carried out using an eluant selected from the group consisting of straight-chain or branched C₁-C₁₀ alkane, C₆-aryl, straight chain or branched C₁-C₆ alkyl alcohol or C₆-aryl alcohol, ammonia, and straight-chain or branched C₁-C₆ alkylamine.

10. A method as claimed in claim 9, in which the straight-chain or branched C₁-C₆ alkyl alcohol is methanol, ethanol, and/or isopropanol.

11. A method as claimed in claim 9, in which C₁-C₁₀ alkane is *n*-heptane.

12. A method as claimed in claim 9 or 10, in which the straight chain or branched C₁-C₆ alkyl alcohol is an aqueous solution

13. A method as claimed in claim 9, in which the straight chain or branched C₁-C₆ alkyl alcohol is an anhydrous preparation.

14. A method as claimed in claim 9, in which the straight chain or branched C₁-C₆ alkyl amine and/or ammonia is an aqueous solution

15. A method as claimed in claim 9, in which the straight chain or branched C₁-C₆ alkyl amine and/or ammonia is formulated as a solution in a straight chain or branched C₁-C₆ alkyl alcohol.

16. A method as claimed in claim 15, in which the solution is methanolic ammonia.

17. A method as claimed in any preceding claim, in which the quantitating the number of naphthenic acid-metal ion complexes present in the eluate is carried out by measuring the UV absorption of the complexes.
